# EUROPEAN PATENT APPLICATION

(11) **EP 3 219 252 A1**
(43) Date of publication of application: **20.09.2017**
(21) Application number: 15859961.3
(22) Date of filing: 07.09.2015
(51) Int. Cl.: A61B 5/022, A61B 5/026, A61B 5/0285

(54) **SKIN PERFUSION PRESSURE MEASUREMENT DEVICE**

(30) Priority: 10.11.2014 JP 2014228319
(71) Applicant: Terumo Kabushiki Kaisha, Tokyo 151-0072 (JP)
(72) Inventor: KURIO, Masaru, Kanagawa 259-0151 (JP)
(74) Representative: Casalonga
(86) International application number: PCT/JP2015/075282
(87) International publication number: WO 2016/076001

(57) **Abstract**

A skin perfusion pressure measuring apparatus measures the skin perfusion pressure of an examined region in a subject. The measuring apparatus includes a measuring device that measures blood flow in the examined region, a detection unit that detects a height of the examined region, and a processing unit that obtains the skin perfusion pressure of the examined region on the basis of a change in the blood flow in the examined region measured by the measuring device with the height of the examined region being changed and the height of the examined region detected by the detection unit.

## Description

### Technical Field

The present invention relates to a measuring apparatus that measures the skin perfusion pressure.

### Background Art

Peripheral blood flow evaluation in peripheral arterial disease is useful, for example, as a method for checking the progress of arteriosclerosis or checking whether there is blood flow required for healing of a wound. There is a method for measuring the skin perfusion pressure as a method of the peripheral blood flow evaluation.

JP2009-506871 W describes a perfusion pressure monitoring system as a skin perfusion pressure measuring apparatus. The skin perfusion pressure monitoring system is provided with an optical probe (sensor), a pressure cuff, and a skin perfusion pressure tool. The optical probe is disposed on the inner face side of the pressure cuff so as to come close to the skin of the limb of a patient. The skin perfusion pressure tool expands the pressure cuff to stop blood flow and then contracts the pressure cuff, and measures the pressure when the blood flow resumes.

In a method that performs pressurization using the pressure cuff like the measuring apparatus described in JP 2009-506871 W, attachment of the pressure cuff to a wound region inflicts pain on a subject. When the pain is excessive, the measurement is difficult to perform. Further, even when pressure is applied to a region other than a wound region using the pressure cuff, pain caused by the pressure may be inflicted on a subject, which is not preferred. Further, when there is calcification in the blood vessel, it may be impossible to stop the blood flow by pressurization.

### Summary of Invention

One aspect of the present invention makes it possible to measure the skin perfusion pressure without applying pressure to an examined region.

One aspect of the present invention relates to a measuring apparatus that measures skin perfusion pressure of an examined region in a subject, and the measuring apparatus includes a measuring device that measures blood flow in the examined region, a detection unit that detects a height of the examined region, and a processing unit that obtains the skin perfusion pressure of the examined region on the basis of a change in the blood flow in the examined region measured by the measuring device with the height of the examined region being changed and the height of the examined region detected by the detection unit.

### Brief Description of Drawings

Fig. 1 is a diagram illustrating a measuring apparatus of one embodiment of the present invention.
Fig. 2 is a diagram illustrating the principle of detecting the height of an examined region in the measuring apparatus of one embodiment of the present invention.
Fig. 3 is a diagram illustrating a usage example of the measuring apparatus of one embodiment of the present invention.
Fig. 4 is a diagram illustrating a measuring method by the measuring apparatus of one embodiment of the present invention.
Fig. 5 is a diagram illustrating a measuring method by the measuring apparatus of one embodiment of the present invention.

### Description of Embodiment

Hereinbelow, the present invention will be described through an exemplary embodiment thereof with reference to the accompanying drawings.

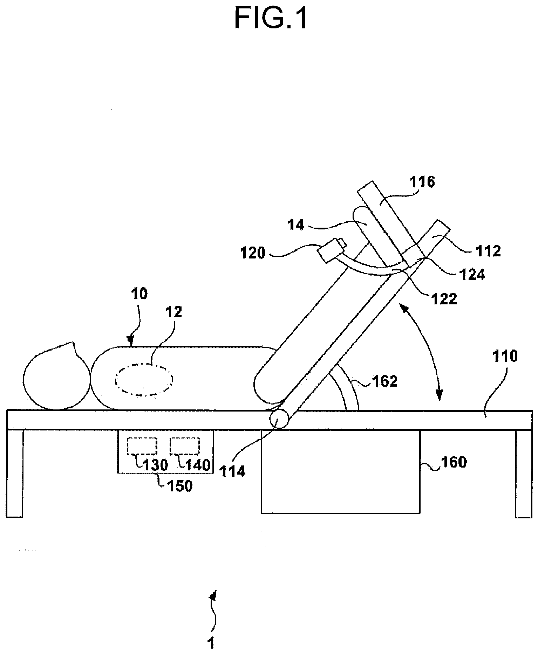

Fig. 1 schematically illustrates a measuring apparatus 1 of one embodiment of the present invention. The measuring apparatus 1 is configured to measure the skin perfusion pressure of an examined region 14 in a subj ect 10. The measuring apparatus 1 measures the skin perfusion pressure using a change in the blood pressure in the examined region 14 by changing the height of the examined region 14. Specifically, the measuring apparatus 1 obtains the skin perfusion pressure of the examined region 14 on the basis of the height of the examined region 14 when the examined region 14 in a state having blood flow is brought into a state with blood flow stopped in response to a change in the height of the examined region 14. Alternatively, the measuring apparatus 1 obtains the skin perfusion pressure of the examined region 14 on the basis of the height of the examined region 14 when the examined region 14 in a state with blood flow stopped is brought into a state with blood flow resumed in response to a change in the height of the examined region 14.

When the specific gravity of mercury is 13.6 and the specific gravity of blood is 1.055, a pressure difference generated by a height difference of 1. 43cm is 1 mmHg. For example, when the heart 12 of the subject 10 is used as a reference, a blood pressure at a position higher than the heart 12 by 1. 43cm becomes lower than a blood pressure at the heart 12 by 1 mmHg. When the examined region 14 is gradually moved upward, the arterial blood pressure at the examined region 14 gradually decreases, and the blood flow may come to a stop in the end. On the contrary, when the examined region 14 lifted to a height where the blood flow in the examined region 14 comes to a stop is gradually moved downward, the blood flow in the examined region 14 resumes in the end.

The measuring apparatus 1 is provided with a measuring device 120 which measures blood flow in the examined region 14, a detection unit 130 which detects the height of the examined region 14, and a processing unit 140. The detection unit 130 and the processing unit 140 may be stored in an electronic component box 150. The detection unit 130 and the processing unit 140 may be configured as one processor or one electronic component.

The detection unit 130 may be configured to detect the height of the examined region 14 relative to the position of the heart 12 (the height where the heart 12 is located) of the subject 10. The processing unit 140 obtains the skin perfusion pressure of the examined region 14 on the basis of a change in the blood flow in the examined region 14 measured by the measuring device 120 with the height of the examined region 14 being changed and the height of the examined region 14 relative to the position of the heart 12, the height being detected by the detection unit 130. The processing unit 140 may be configured to obtain the skin perfusion pressure of the examined region 14 on the basis of a value obtained by converting the height of the examined region 14 relative to the position (height) of the heart 12 of the subject 10 into a blood pressure (arterial blood pressure).

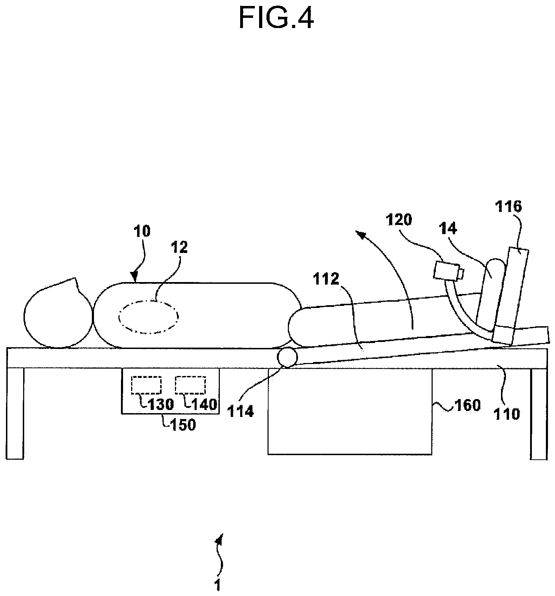

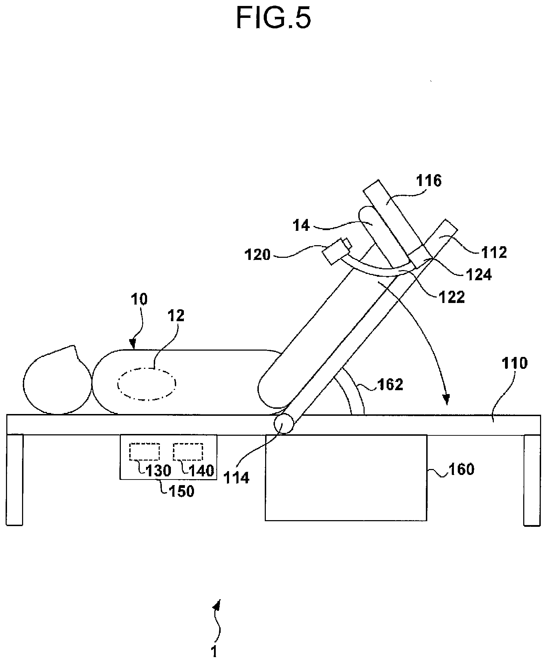

As illustrated in Fig. 4, the processing unit 140 acquires the height of the examined region 14 when blood flow in the examined region 14 comes to a stop in response to an upward movement of the examined region 14 from the detection unit 130 and obtains the skin perfusion pressure of the examined region 14 on the basis of the acquired height. Alternatively, as illustrated in Fig. 5, the processing unit 140 acquires the height of the examined region 14 when the blood flow in the examined region 14 resumes in response to a downward movement of the examined region 14 from the detection unit 130 and obtains the skin perfusion pressure of the examined region 14 on the basis of the acquired height.

The measuring apparatus 1 may further be provided with a change mechanism 160 which changes the height of the examined region 14. The change mechanism 160 may automatically change the height of the examined region 14, or may change the height of the examined region 14 in response to an operation by a measurer or by the force of a measurer. In an example, the measuring apparatus 1 is provided with a fixed table 110 which supports the subject 10 in a lying posture and a movable table 112 which supports the limb (e . g. , the leg) of the subject 10. The movable table 112 may be turnably supported by a pivotal support member 114 which is attached to the fixed table 110. The change mechanism 160 may be configured to drive the movable table 112 by an actuator (e.g., a motor) through a movable arm 162.

A fixing unit 116 which fixes the toe which may be the examined region 14 is attached to the movable table 112. The fixing unit 116 may be configured to be capable of adjusting the angle thereof with respect to the movable table 112. The measuring apparatus 1 may further be provided with a holding unit 122 which holds the measuring device 120 so as to maintain a relative position between the measuring device 120 and the examined region 14 when the height of the examined region 14 is being changed.

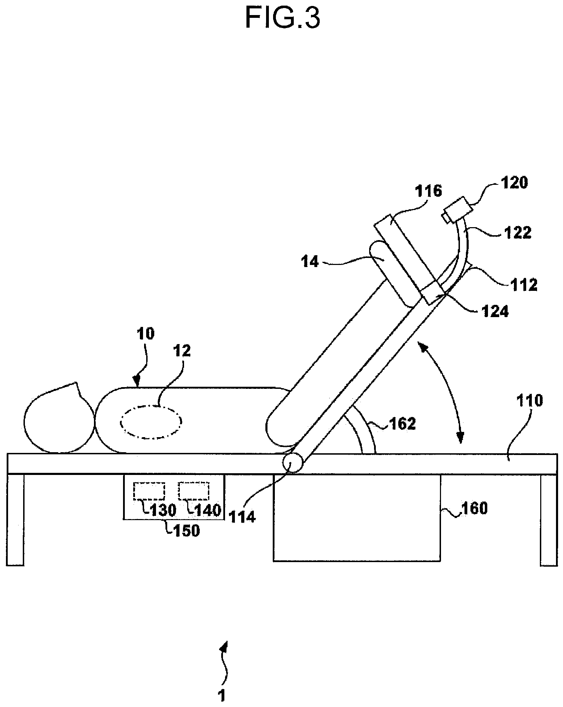

The holding unit 122 may include an attitude changing mechanism 124 which changes the attitude of the measuring device 120 so that the examined region 14 comes into a measurable area for the measuring device 120. As can be understood from a comparison between Figs. 1 and 3, the attitude changing mechanism 124 may include a mechanism which turns the holding unit 122 holding the measuring device 120. In Fig. 1, the instep of the foot as the examined region 14 is observed (measured) by the measuring device 120. In Fig. 3, the sole of the foot as the examined region 14 is observed (measured) by the measuring device 120. The fixing unit 116 maybe configured to allow the measuring device 120 to measure blood flow in the examined region 14 through the fixing unit 116. Specifically, the fixing unit 116 may be configured to transmit light for measurement. This is achieved, for example, by the fixing unit 116 composed of a material that transmits light or the fixing unit 116 having a structure including an opening so as not to cover the examined region 14. As illustrated in Fig. 3 as an example, when the eyes of the subject 10 are located within the field of view of the measuring device 120 and the measuring device 120 emits light such as laser light, a light blocking member may be disposed between the eyes of the subject 10 and the examined region 14.

The measuring device 120 maybe configured to measure blood flow at a plurality of positions in the examined region 14 of the subject 10. The processing unit 140 may be configured to generate an image representing a skin perfusion pressure distribution in the examined region 14 on the basis of changes in the blood flow at the plurality of positions in the examined region 14 measured by the measuring device 120. The generated image may be output to an output unit (e.g., a display unit or a printer, not illustrated). For example, a laser Doppler blood flow imaging device such as PeriScan PIM III provided by Perimed AB can be applied as the measuring device 120. Alternatively, a laser speckle blood flowmeter such as Laser Speckle Contrast Analysis (LASCA) provided by Perimed AB can be applied as the measuring device 120. In addition, a plurality of probes which are brought into intimate contact with the examined region 14 to measure blood flow may be provided.

The stoppage or the resumption of blood flow in the examined region 14 may be determined when the stoppage or the resumption of blood flow has been observed (measured) by the measuring device 120 in an area having a predetermined ratio to the entire area of the examined region 14 or when the stoppage or the resumption of blood flow has been observed (measured) by the measuring device 120 at a predetermined number of positions which are preset inside the examined region 14. The measuring device 120 may be configured to measure blood flow at one specific position in the examined region 14.

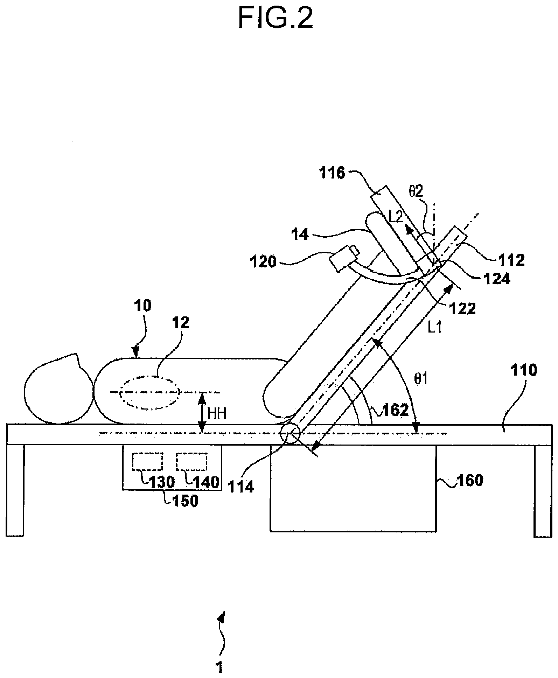

The principle of detecting the height of the examined region 14 by the detection unit 130 will be described as an example with reference to Fig. 2. The height of the examined region 14 from the pivotal support member 114 is represented by L1sinθ1 + L2cosθ2, where L1 denotes the distance from the center of the pivotal support member 114 to a coupling site between the movable table 112 and the fixing unit 116, θ1 denotes the angle between the fixed table 110 and the movable table 112, L2 denotes the distance from the movable table 112 at the fixing unit 116, and θ2 denotes the angle between the fixing unit 116 and a vertical plane. The distance L2 can be specified from coordinates within the field of view of the measuring device 120. When a height difference HH between the pivotal support member 114 and the heart 12 is taken into consideration, the height of the examined region 14 relative to the heart 12 is represented by L1sinθ1 + L2cosθ2 - HH.

The distance L1, the angle θ1, and the angle θ2 may be provided to the detection unit 130 from a sensor which reads these L1, θ1, and θ2 or may be input to the detection unit 130 by a measurer. The distance L2 may be specified on the basis of a position in information (image) output from the measuring device 120. Note that the information (image) output from the measuring device 120 or the height in the field of view of the measuring device 120 may be considered to be constant.

Alternatively, the detection unit 130 may detect the height of the examined region 14 on the basis of a measurement result provided from a sensor (not illustrated) which measures the height of the examined region 14. The sensor may be, for example, a laser displacement meter.

Although, in the present embodiment, the height of the examined region 14 is changed by moving the movable table 112 up and down around the pivotal support member 114 as a rotation shaft with respect to the fixed table 110 whose height is fixed, the present invention is not limited thereto. The position of the heart 12 of the subject 10 may be moved up and down by moving the fixed table 110 up and down around the pivotal support member 114 as a rotation shaft without changing the position of the movable table 112 to change the relative height between the examined region 14 and the heart 12. Further, it is also possible to change the relative position between the examined region 14 and the heart 12 by changing both the position of the fixed table 110 and the position of the movable table 112.

The present invention is not limited to the above embodiment, and various changes and modifications can be made without departing from the scope and the range of the invention. Thus, the following claims are appended to make the range of the invention public.

This application claims priority to Japanese Patent Application No. 2014-228319 filed on November 10, 2014, the disclosure of which is incorporated herein by reference in its entirely.

### Reference Signs List

- 10:: subject
- 12:: heart
- 14:: examined region
- 110:: fixed table
- 112:: movable table
- 116:: fixing unit
- 120:: measuring unit
- 130:: detection unit
- 140:: processing unit
- 160:: change unit

## Claims

1. A measuring apparatus that measures skin perfusion pressure of an examined region in a subject, the measuring apparatus comprising:
a measuring device that measures blood flow in the examined region;
a detection unit that detects a height of the examined region; and
a processing unit that obtains the skin perfusion pressure of the examined region on the basis of a change in the blood flow in the examined region measured by the measuring device with the height of the examined region being changed and the height of the examined region detected by the detection unit.

2. The measuring apparatus according to claim 1, wherein the detection unit detects the height of the examined region relative to a position of the heart of the subject.

3. The measuring apparatus according to claim 2, wherein the processing unit obtains the skin perfusion pressure of the examined region on the basis of a value obtained by converting the height of the examined region relative to the position of the heart of the subject into a blood pressure.

4. The measuring apparatus according to any one of claims 1 to 3, wherein the processing unit acquires the height of the examined region when the blood flow in the examined region comes to a stop in response to relative movement of the examined region to a position above the heart or when the blood flow in a stopped state in the examined region resumes in response to relative movement of the examined region to a position closer to the heart, from the detection unit, and obtains the skin perfusion pressure of the examined region on the basis of the height.

5. The measuring apparatus according to any one of claims 1 to 4, further comprising a change mechanism that changes a relative height between the examined region and the heart.

6. The measuring apparatus according to any one of claims 1 to 5, wherein
the measuring device measures blood flow at a plurality of positions in the examined region of the subject, and
the processing unit generates an image representing a skin perfusion pressure distribution in the examined region on the basis of changes in the blood flow at the plurality of positions in the examined region measured by the measuring device.

7. The measuring apparatus according to any one of claims 1 to 6, further comprising a holding unit that holds the measuring device so as to maintain a relative position between the measuring device and the examined region when the height of the examined region is being changed.

8. The measuring apparatus according to claim 7, wherein the holdingunit includes an attitude changingmechanismthat changes attitude of the measuring device so that the examined region comes into a measurable area for the measuring device.

9. The measuring apparatus according to any one of claims 1 to 8, further comprising a fixing unit that fixes the examined region.

10. The measuring apparatus according to claim 9, wherein the fixing unit is configured to allow the measuring device to measure the blood flow in the examined region through the fixing unit.
